(19) Europäisches Patentamt
European Patent Office
Office européen des brevets

(11) EP 4 428 131 B1

(12) # EUROPEAN PATENT SPECIFICATION

(45) Date of publication and mention of the grant of the patent:
**08.04.2026 Bulletin 2026/15**

(21) Application number: **22904670.1**

(22) Date of filing: **07.12.2022**

(51) International Patent Classification (IPC):
***C07F 9/6561*** (2006.01) ***C07D 405/14*** (2006.01)
***A61K 31/4184*** (2006.01) ***A61P 1/04*** (2006.01)
***C07D 405/04*** (2006.01) ***A23L 33/105*** (2016.01)
***C07F 9/6558*** (2006.01) ***A23L 33/10*** (2016.01)
***A61K 31/675*** (2006.01) ***A61K 31/683*** (2006.01)
***A61K 31/685*** (2006.01)

(52) Cooperative Patent Classification (CPC):
**C07D 405/14; A23L 33/10; A61K 31/4184; A61K 31/675; A61K 31/683; A61K 31/685; A61P 1/04; C07D 405/04; C07F 9/65586; C07F 9/6561**

(86) International application number:
**PCT/KR2022/019846**

(87) International publication number:
**WO 2023/106841 (15.06.2023 Gazette 2023/24)**

(54) **BENZIMIDAZOLE DERIVATIVE COMPOUND AND USES THEREOF**
BENZIMIDAZOLDERIVATVERBINDUNG UND VERWENDUNGEN DAVON
COMPOSÉ DÉRIVÉ DE BENZIMIDAZOLE ET SES UTILISATIONS

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **08.12.2021 KR 20210174301**

(43) Date of publication of application:
**11.09.2024 Bulletin 2024/37**

(73) Proprietor: **Pharmgen Science, Inc.**
**Hwaseong-si, Gyeonggi-do 18622 (KR)**

(72) Inventors:
- **YEO, Marie**
**Yongin-si Gyeonggi-do 16948 (KR)**
- **OH, Je Do**
**Yongin-si Gyeonggi-do 16827 (KR)**
- **LEE, Seul Ae**
**Seongnam-si Gyeonggi-do 13644 (KR)**
- **LEE, Sang Hak**
**Hwaseong-si Gyeonggi-do 18497 (KR)**
- **KIM, Hye Youn**
**Seoul 06092 (KR)**
- **PARK, Hee Deok**
**Hwaseong-si Gyeonggi-do 18482 (KR)**
- **HAN, Eui Sang**
**Seoul 06547 (KR)**
- **KIM, Dong Kyu**
**Suwon-si Gyeonggi-do 16694 (KR)**
- **LEE, Do Hyung**
**Suwon-si Gyeonggi-do 16287 (KR)**
- **KANG, Seung Hee**
**Bucheon-si Gyeonggi-do 14574 (KR)**

(74) Representative: **Isarpatent**
**Patent- und Rechtsanwälte**
**Barth Hassa Peckmann & Partner mbB**
**Friedrichstraße 31**
**80801 München (DE)**

(56) References cited:
**WO-A1-2006/134460**
**WO-A1-2020/210878**
**KR-A- 20120 052 269**
**KR-B1- 101 088 247**
**KR-B1- 101 829 706**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

• ABET VALENTINA ET AL: "Prodrug approach: An overview of recent cases", EUROPEAN JOURNAL OF MEDICINAL CHEMISTRY, ELSEVIER MASSON, AMSTERDAM, NL, vol. 127, 29 October 2016 (2016-10-29), pages 810 - 827, XP029907336, ISSN: 0223-5234, DOI: 10.1016/ J.EJMECH.2016.10.061

## Description

### [Technical Field]

The invention relates to a benzimidazole derivative compound and use thereof, more specifically to the following the benzimidazole derivative compound represented by Formula 1 or Formula 2, or a pharmaceutically acceptable salt thereof or a stereoisomer thereof and use thereof:

[Formula 1]

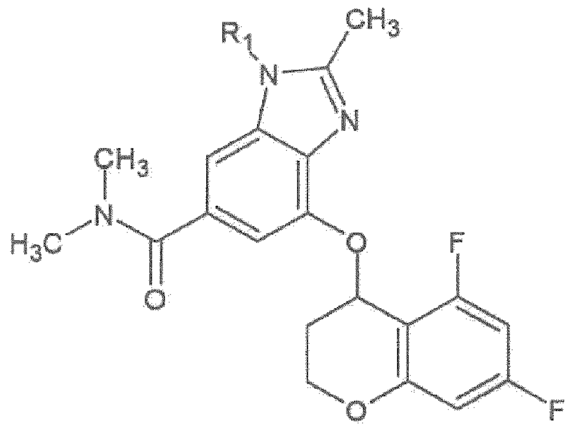

[Formula 2]

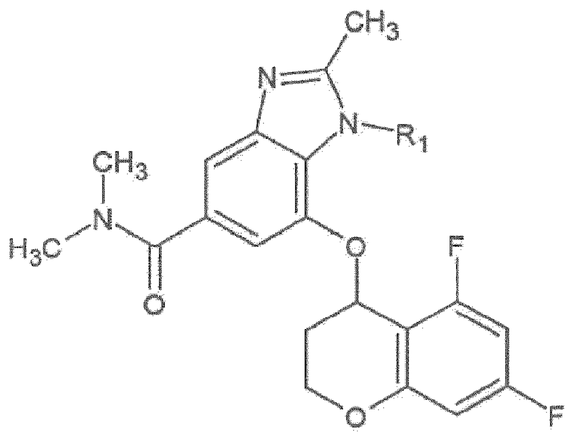

in the Formula above,
wherein $R_1$ is the same as defined in the specification.

### [Background Art]

A gastric acid plays a positive role in digesting proteins, absorbing minerals such as calcium and iron, and sterilizing harmful microorganisms in ingested food. However, because gastric acid is a very strong acid with a pH of around 1 to 2, excessive gastric acid secretion or reflux into the esophagus can cause gastritis, gastric ulcers, and gastroesophageal reflux disease (GERD) .

The gastric acid secretion is made with the proton pump (H+/K+-ATPase) of parietal cells existing in the gastric mucosa. Due to various gastric acid secretion stimulating factors, the proton pump consumes ATP and excretes hydrogen ions into the gastric cavity by exchanging hydrogen ions in the cytoplasm and potassium ions in the stomach lumen.

As drugs to suppress gastric acid secretion, H2 receptor blockers(H2 receptor antagonists, H2 RAs) and proton pump inhibitors(PPIs) have been commonly used. However, H2 RA does not directly inhibit the proton pump, but only blocks the histamine pathway that stimulates gastric acid secretion, so it has a partial effect. In addition, PPIs are prodrugs and must be taken before meals because they exert their effects after being activated by stomach acid, Because the onset

of action is slow and the half-life is short, there are limitations in suppressing the symptoms of nocturnal acid breakthrough (NAB). Therefore, there is a need to develop drugs that inhibit gastric acid secretion with a new mechanism that can compensate for these shortcomings.

Recently, vonoprazan (Japan, 2015) and tegoprazan (Korea, 2019) were released as a new class of gastric acid suppressing drugs, P-CAB (potassium-competive acid blocker). P-CAB class drugs have a 5 to 100-fold improvement in the ability to inhibit the proton pump that secretes gastric acid compared to existing PPIs, demonstrating stronger efficacy than PPIs. In addition, in the mechanism of action, existing PPIs are precursors (prodrugs) and require an activation process in acid, but P-CAB does not require an activation process. In particular, while PPI has an irreversible covalent bond with the proton pump, P-CAB has the advantage of having a very fast onset time, a long duration of action, and being able to be taken regardless of diet by bonding reversibly.

However, among the P-CAB class drugs, tegoprazan (Korea, 2019) has pH-dependent water solubility and is a poorly soluble drug with significantly low solubility, especially in neutral aqueous solutions. Due to these characteristics, when the pH in the gastric cavity rises to neutral, such as during a meal, the solubility of the drug decreases, which reduces bioavailability and makes it difficult to formulate various formulations such as neutral injections.

Abet Valentina et al., "Prodrug approach: An overview of recent cases", European Journal of Medicinal Chemistry, 127, 2016, pp. 810-827 discusses modern trends in prodrug strategy, providing selected examples and classifying them according to the aim of their designs. WO 2006/134460 A1 discloses compounds for use in the treatment of a condition mediated by acid pump antagonistic activity. KR 2012 0052269 A and KR 101 088 247 B1 relate to the use of compounds having acid pump antagonist activity, and their pharmaceutically acceptable salt.KR 101 829 706 B1 discloses acid addition salts of S-4-57-difluorochroman-4-yloxy-NN2-trimethyl-1H-benzo[d]imidazole-6-carboxamide and their use for preventing and treating diseases mediated by acid pump antagonistic activities, as well as the production thereof. WO 2020/210878 A1 is directed to pharmaceutical combinations, compositions and kits comprising at least one H+/K+ ATPase inhibitor and an antibiotic compound and methods of use thereof in the treatment, prevention and amelioration of Helicobacter pylori infections in an individual.

**[Disclosure]**

**[Technical Problem]**

The purpose of the present invention is to provide a novel benzimidazole derivative compound represented by Formula 1 or Formula 2 that exhibits gastric acid secretion inhibition activity.

Another object of the present invention is to provide a pharmaceutical composition for inhibiting gastric acid secretion, comprising the above compound as an active ingredient.

Another object of the present invention is to provide a pharmaceutical composition for preventing or treating diseases caused by gastric acid secretion disorders, comprising the above compound as an active ingredient.

**[Technical Solution]**

According to one aspect of the present invention, a benzimidazole derivative compound represented by Formula 1 or Formula 2, or a pharmaceutically acceptable salt thereof or a stereoisomer thereof provided:

[Formula 1]

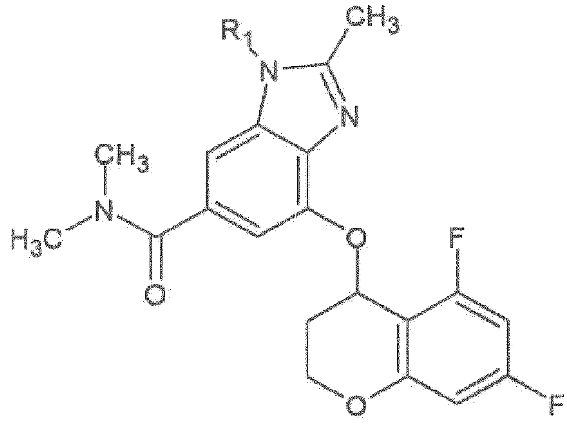

[Formula 2]

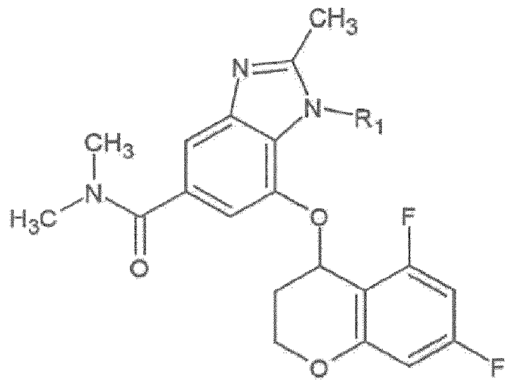

in the Formula above,

wherein $R_1$ is

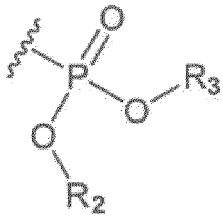 , 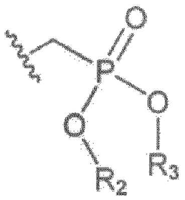 or 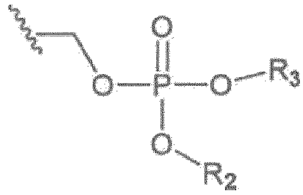 ,

wherein $R_2$ and $R_3$ are each independently hydrogen, alkyl or cycloalkyl.

The benzimidazole derivative compound of Formula 1 or Formula 2 according to the present invention can form a pharmaceutically acceptable salt. The pharmaceutically acceptable salts may include, but are not limited to, alkali metal or alkaline earth metal salts formed by, for example, sodium, lithium, potassium, calcium, magnesium, etc. The benzimidazole derivative compound of Formula 1 or Formula 2 according to the present invention can be converted into its salt by a conventional method.

In the meantime, the compounds according to the present invention may have an asymmetric carbon center and therefore may exist as R or S isomers, racemates, diastereomeric mixtures and individual diastereomers, and all of these isomers and mixtures are included in the scope of the present invention.

In this specification, unless otherwise specified for convenience, the benzimidazole derivative compound of Formula 1 or Formula 2 is used to include the compound of Formula 1 or Formula 2, and pharmaceutically acceptable salts and stereoisomers thereof.

In defining the benzimidazole derivative compound of Formula 1 or Formula 2 throughout this specification, the following concepts defined for substituents are used.

Unless otherwise stated, the term "alkyl" herein refers to a radical the saturated aliphatic hydrocarbon group having the straight-chain or the branched, for example, the carbon atom of 1 to 7 in the present application. As an example of the alkyl group, methyl, ethyl, n-propyl, isopropyl, n-butyl, isobutyl, sec-butyl, tert-butyl, n-pentyl, isopentyl, neopentyl, tert-pentyl, 1-methylbuthyl, 2-methylbuthyl, 1-ethylpropyl and 1,2-dimethylpropyl etc. are included but it is not limited thereto.

Unless otherwise stated, the term "cycloalkyl" herein refers to a radical of the group of cyclic, for example, saturated aliphatic hydrocarbons having 3 to 7 carbon atoms. As an example of the cycloalkyl group, cyclopropyl, cyclobutyl, cyclopentyl, cyclohexyl etc is included but it is not limited thereto.

According to one embodiment of the invention, in Formula 1 or Formula 2, $R_2$ and $R_3$ may each independently be hydrogen, $C_1$-$C_7$ alkyl or $C_3$-$C_7$ cycloalkyl.

According to another embodiment of the present invention, in Formula 1 or Formula 2, $R_2$ and $R_3$ may each independently be hydrogen or $C_1$-$C_5$ alkyl.

Representative benzimidazole derivative compounds of Formula 1 or Formula 2 according to the present invention may include, but are not limited to, the following compounds:

diisopropyl(S)-(4-((5,7-difluorochroman-4-yl)oxy)-6-(dimethylcarbamoyl)-2-methyl-1H-benzo[d]imidazol-1-yl)phosphonate;
(S)-di-tert-butyl((4-((5,7-difluorochroman-4-yl)oxy)-6-(dimethylcarbamoyl)-2-methyl-1H-benzo[d]imidazol-1-yl) methyl) phosphate;
tert-butyl((4-(((S)-5,7-difluorochroman-4-yl)oxy)-6-(dimethylcarbamoyl)-2-methyl-1H-benzo[d]imidazol-1-yl) methyl)hydrogen phosphate;
(S)-(4-((5,7-difluorochroman-4-yl)oxy)-6-(dimethylcarbamoyl)-2-methyl-1H-benzo[d]imidazol-1-yl)methyl dihydrogen phosphate;
sodium(S)-tert-butyl ((4-((5,7-difluorochroman-4-yl)oxy)-6-(dimethylcarbamoyl)-2-methyl-1H-benzo[d]imidazol-1-yl)methyl) phosphate;
sodium(S)-(4-((5,7-difluorochroman-4-yl)oxy)-6-(dimethylcarbamoyl)-2-methyl-1H-benzo[d]imidazol-1-yl)methyl phosphate.

According to another aspect of the present invention, a pharmaceutical composition for the inhibition of gastric acid secretion comprising the benzimidazole derivative compound of the Formula 1 or Formula 2, or a pharmaceutically acceptable salt thereof or a stereoisomer thereof as an active ingredient, with a pharmaceutically acceptable carrier provided.

According to another aspect of the present invention, a pharmaceutical composition for the preventing or treating diseases caused by disorders of gastric acid secretion comprising the benzimidazole derivative compound of the Formula 1 or Formula 2, or a pharmaceutically acceptable salt thereof or a stereoisomer thereof as an active ingredient, with a pharmaceutically acceptable carrier provided.

In the present invention, "pharmaceutical composition" may include other chemical components such as carriers, diluents, excipients, etc. in addition to the active compound according to the present invention. Accordingly, the pharmaceutical composition may include a pharmaceutically acceptable carrier, diluent, excipient, or a combination thereof, if necessary.

According to another embodiment of the present invention, the diseases caused by gastric acid secretion disorders include gastrointestinal diseases, gastroesophageal diseases, gastroesophageal reflux disease (GERD), peptic ulcers, gastric ulcers, duodenal ulcers, NSAID-induced ulcers, gastritis, and Helicobacter pylori. Helicobacter pylori infection, indigestion, functional dyspepsia, Zollinger-Ellison syndrome, non-erosive reflux disease (NERD), visceral pain, heartburn, nausea, esophagitis, dysphagia, drooling, airway disorder, or asthma, but is not limited thereto.

**[Advantageous Effects]**

Since the benzimidazole derivative compound represented by Formula 1 or Formula 2 according to the present invention exhibits an effect of suppressing gastric acid secretion, it can function as a potassium-competitive acid blocker (P-CAB) and inhibits gastric acid secretion. It can be used as a secretion inhibitor or as a preventive or treatment agent for diseases caused by gastric acid secretion disorders. In addition, the benzimidazole derivative compound represented by Formula 1 or Formula 2 according to the present invention has significantly improved neutral water solubility than tegoprazan, which was previously developed as a gastric acid suppressing drug, and is not affected by pH in vivo, such as diet, and it is facilitated in the formulation including the injection agent.

**[Mode for Carrying Out the Invention]**

Hereinafter, the present invention will be described in more detail through preparation examples and examples. However, these examples are only illustrative of the present invention, and the scope of the present invention is not limited thereto.

An explanation of the abbreviations used in the examples below is as follows:

ACN: acetonitrile.
DMF: N,N- dimethylformamid.
EtOAc: ethyl acetate.
FA: formic acid
MeOH: methanol.

**<u>embodiment 1: Synthesis of diisopropyl (S)-(4-((5,7-difluorochroman-4-yl)oxy)-6-(dimethylcarbamoyl)-2-methyl-1H-benzo(d)imidazol-1-yl)phosphonate</u>**

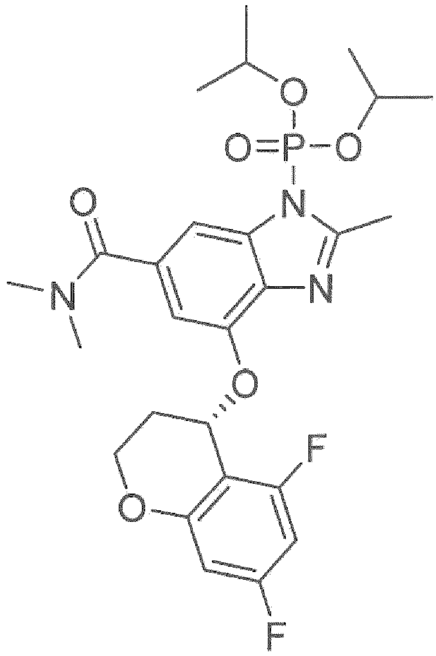

7-[[(4S)-5,7-difluoro-3,4-dihydro-2H-chromen-4-yl]oxy]-N,N,2-trimethyl-3H-benzimidazole-5-Carboxamide (80mg, 0.21mmol) was dissolved in dry DMF(2mL), and then 60% NaH(12.39mg, 0.31mmol) was added at 0°C under a nitrogen atmosphere and stirred at room temperature for 30 minutes. Diisopropyl phosphorochloridate (53.85mg, 0.27mmol) was added to this reaction at 0°C and stirred at room temperature for 1 hour. After completion of the reaction, it was extracted with EtOAc, washed with brine, dried with anhydrous Na2SO4, and separated by column chromatography (EtOAc:Hexane:MeOH = 6:6:1) to obtain the target compound, diisopropyl (S)-(4-((5,7-difluorochroman-4-yl)oxy)-6-(dimethylcarbamoyl)-2-methyl-1H-benzo[d]imidazole - 1-yl)phosphonate (43mg, 37%) as a white solid.

NMR(DMSO-d6): 7.13 (1H), 6.93(s, 1H), 6.83(t, 1H), 6.70(d, 1H), 6.03(s, 1H), 4.43~4.37(m, 3H), 4.25(t, 1H), 2.97(s, 6H), 2.45(s, 3H), 2.24(d, 1H), 2.07(t, 1H), 1.24 (d, 12H); MS+:552.44

**embodiment 2: Synthesis of (S)-di-tert-butyl((4-((5,7-difluorochroman-4-yl)oxy)-6-(dimethylcarbamoyl)-2-methyl-1H-benzo[d]imidazol-1-yl)methyl) phosphate**

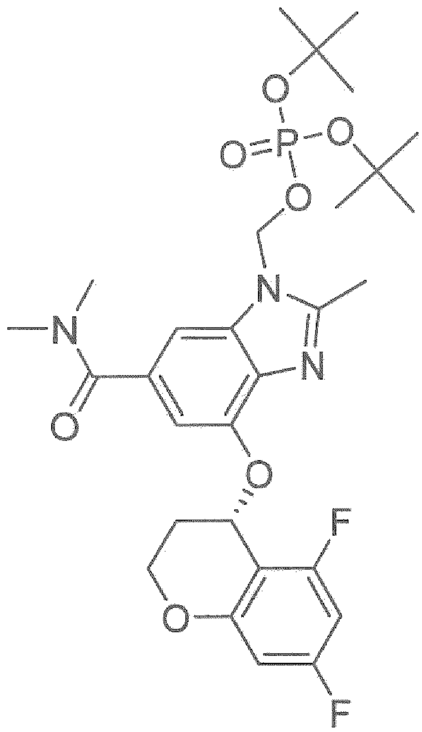

7-[[(4S)-5,7-difluoro-3,4-dihydro-2H-chromen-4-yl]oxy]-N,N,2-trimethyl-3H-benzimidazole-5 -Carboxamide (1,000mg, 2.58mmol) was dissolved in dry DMF (25.8mL), and then 60% NaH (154.89mg, 3.87mmol) was added at 0°C under a nitrogen atmosphere and stirred at room temperature for 30 minutes. Diisopropyl phosphorochloridate (1,336mg, 5.16mmol) was added to this reaction at 0°C and stirred at room temperature for 1 hour. After completion of the reaction, it was extracted with EtOAc, washed with brine, dried with anhydrous Na2SO4, and separated by column chromatography (EtOAc:Hexane:MeOH = 6:6:1) to obtain the target compound, (S)-di-tert-butyl(4-((5,7-difluorochroman-4-yl)oxy)-6-(dimethylcarbamoyl)-2-methyl-1H-benzo[d]imidazol-1-yl)methyl)Phosphate (812mg, 52%) was obtained as a white solid.

NMR(DMSO-d6): 7.30(s, 1H), 6.94(s, 1H), 6.81(t, 1H), 6.76(d, 1H), 6.14(s, 1H), 6.02(d, 2H), 4.36(d, 1H), 4.22(d, 1H), 2.97(s, 6H), 2.61(s, 3H), 2.22(d, 1H), 2.07(t, 1H), 1.38~1.26(m, 18H); MS+: 610.33

**embodiment 3: Synthesis of tert-butyl ((4-(((S)-5, 7-difluorochroman-4-yl)oxy)-6-(dimethylcarbamoyl)-2-methyl-1H-benzo(d)imidazol-1-yl)methyl)hydrogen phosphate**

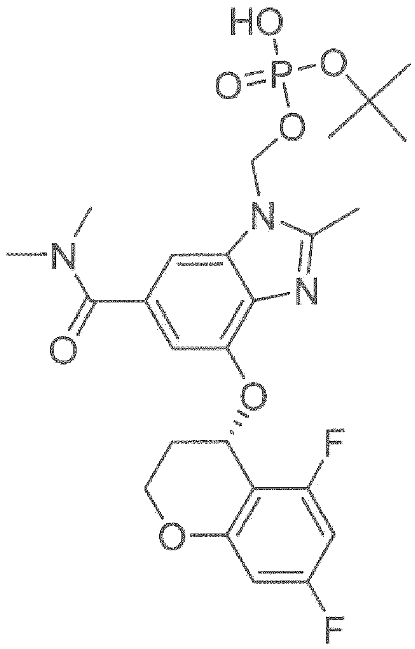

(S)-di-tert-butyl(4-((5,7-difluorochroman-4-yl)oxy)-6-(dimethylcarbamoyl)-2-methyl-1H-benzo[d] Imidazol-1-yl) methyl)phosphate (295 mg, 0.49 mmol) was dissolved in dry DMF (4.86 mL), and then 4N HCl (1.4 mL) was added at 0°C and stirred at room temperature for 90 minutes. After completion of the reaction, it was separated by reversed-phase column chromatography (0.1% FA) and freeze-dried, to obtain the target compound, tert-butyl((4-(S)-5,7-difluorochroman-4-yl)oxy-6-(dimethylcarbamoyl)-2-methyl-1H-benzo[d]imidazol-1-yl)methyl)hydrogen phosphate (74.2 mg, 27%) was obtained as a white solid.

NMR(DMSO-d6): 7.31(s, 1H), 6.94(s, 1H), 6.81(t, 1H), 6.71(d, 1H), 6.15(s, 1H), 5.96(d, 2H), 4.37(d, 1H), 4.23(t, 1H), 2.98(s, 6H), 2.64(s, 3H), 2.24(d, 1H), 2.07(m, 1H), 1.26(s, 9H); MS+:554.47

**embodiment 4: Synthesis of (S)-(4-((5,7-difluorochroman-4 -yl)oxy)-6-(dimethylcarbamoyl)-2-methyl-1H-benzo [d]imidazol-1-yl)methyl dehydrogen phosphate**

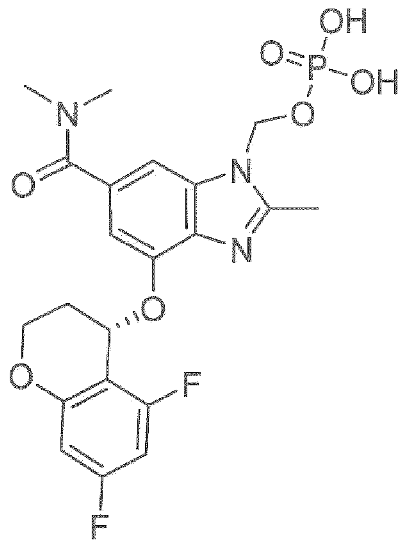

Tert-butyl((4-(S)-5,7-difluorochroman-4-yl)oxy)-6-(dimethylcarbamoyl)-2-methyl-1H-benzo[d]imidazole- 1-yl) methyl)hydrogen phosphate (200 mg, 0.33 mmol) was dissolved in DMF (6.57 mL), then 4N HCl (0.5 mL) was added at 0°C and stirred at room temperature for 90 minutes. After completion of the reaction, it was separated by reverse-phase column chromatography (0.1% FA) and lyophilized to obtain the target compound, (S)-(4-((5,7-difluorochroman-4-yl)oxy)-6-(dimethylcarbamoyl)-2-methyl-1H-benzo[d]imidazol-1-yl)methyl dihydrogen phosphate (64.1 mg, 35%) was obtained as a white solid.

NMR(DMSO-d6): 7.31(s, 1H), 6.94(s, 1H), 6.81(t, 1H), 6.71(d, 1H), 6.14(s, 1H), 5.95(d, 2H), 4.37(d, 1H), 4.22(t, 1H), 2.98(s, 6H), 2.64(d, 3H), 2.25(d, 1H), 2.07(m, 1H); MS+:498.29

**embodiment 5: Synthesis of sodium (S)-tert-butyl((4-((5,7-difluorochroman-4-yl)oxy)-6-(dimethylcarbamoyl)-2-methyl-1H-benzo(d)imidazol-1-yl)methyl)phosphate)**

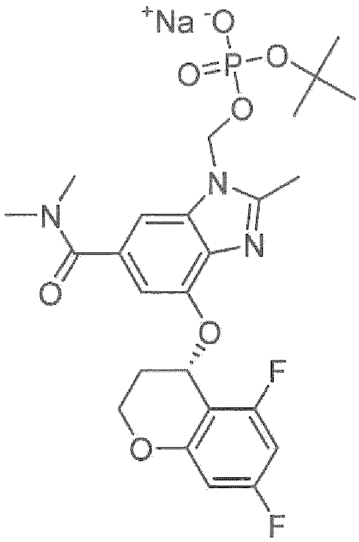

Tert-butyl((4-(S)-5,7-difluorochroman-4-yl)oxy)-6-(dimethylcarbamoyl)-2-methyl-1H-benzo[d]imidazole- 1-yl) methyl)hydrogen phosphate (52.8 mg, 0.095 mmol) was dissolved in ACN:H2O = 1:1 (9.5 mL), then NaOH (4.2mg, 0.105 mmol) was added and stirred at room temperature for 1 minute. After completion of the reaction, it was lyophilized to obtain the target compound, sodium (S)-tert-butyl(4-((5,7-difluorochroman-4-yl)oxy)-6-(dimethylcarbamoyl)-2-Methyl-1H-benzo[d]imidazol-1-yl)methyl)phosphate (54 mg, 98%) was obtained as a white solid.

NMR(D20): 7.47(s, 1H), 7.13(s, 1H), 6.65~6.56(m, 2H), 5.96~5.93(m, 3H), 4.51~4.45(m, 2H), 3.15(s, 3H), 3.05(s, 3H), 2.72(s, 3H), 2.39(d, 1H), 2.22~2.14(m, 1H), 1.04(s, 9H); MS+: 554.35

**embodiment 6: Synthesis of sodium (S)-(4-((5,7-difluorochroman-4-yl)oxy)-6-(dimethylcarbamoyl)-2-methyl-1H-benzo(d)imidazol-1-yl)methyl phosphate)**

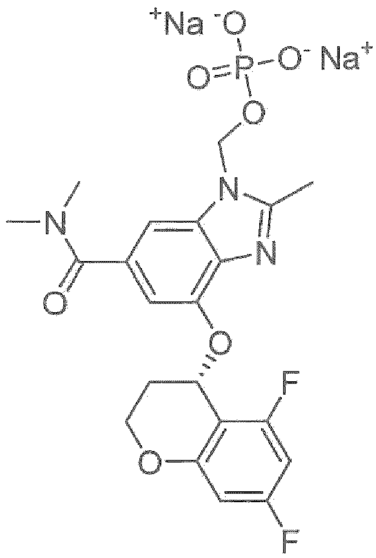

(S)-(4-((5,7-difluorochroman-4-yl)oxy)-6-(dimethylcarbamoyl)-2-methyl-1H-benzo[d]imidazole-1-1)methyl dihydrogen phosphate (51.6 mg, 0.104 mmol) was dissolved in ACN:H2O=1:1(10.4 mL), then NaOH(8.7 mg, 0.218 mmol) was added and stirred at room temperature for 1 minute. After completion of the reaction, it was lyophilized to obtain the target compound, sodium (S)-(4-((5,7-difluorochroman-4-yl)oxy)-6-(dimethylcarbamoyl)-2-methyl -1H-benzo[d]imidazol-1-yl)methyl phosphate (52 mg, 93%) was obtained as a white solid.

NMR(D2O): 7.52(s, 1H), 7.11 (s, 1H), 6.65~6.58(m, 2H), 5.95(s, 1H), 5.84(d, 2H), 4.48~4.45(m, 2H), 3.15(s, 3H), 3.06(s, 3H), 2.71(s, 3H), 2.41(d, 1H), 2.22~2.13(m, 1H); MS+: 498.33

**experimental example 1: water solubility test (pH 6.8).**

After it measured so that each testing material was concentrated by 5.12 mg/ml in the glass vial, 0.5M Tris-HCl (pH 6.8, T&I, BTH-9168) was added and it processed in for one minute mixing, and treated in a sonicator for 30 minutes the solution was prepared. The solution was observed with the naked eye and recorded in photographs. If it was not completely dissolved, additional buffer was added to prepare a solution and the observation was repeated. Solubility was evaluated by checking the concentration when completely dissolved. Tegoprazan was used as a comparison compound. The measured results are shown in Table 1 below.

[Table 1]

| Compound | Solubility, pH 6.8(mg/ml) |
|---|---|
| Comparison compound | 0.02 |
| Embodiment 1 | 0.01 |
| Embodiment 2 | 5.12 |
| Embodiment 3 | 2.56 |
| Embodiment 4 | 2.56 |
| Embodiment 5 | 5.12 |
| Embodiment 6 | 5.12 |

As shown in Table 1, the compounds of Examples 3 and 4 according to the present invention showed 128 times improved water solubility (pH 6.8) compared to the comparative compounds, and Examples 2, 5, and The compound of Example 6 showed 256 times improved water solubility (pH 6.8) compared to the comparative compound. Therefore, the new benzimidazole derivative compound of the present invention, which has significantly improved water solubility, has no effect on solubility even when the pH in the gastric cavity increases due to diet, and furthermore, it can be easily formulated, including injections.

**experimental example 2: gastric acid releasing suppression ability qualification test by the oral administration.**

A week before the test, test animals (rats) were purchased and acclimatized. After acclimatization, food was removed the day before the test and fasting was performed for 24 hours using a fasting plate. The weight of each individual was measured, weight ranking was sequentially assigned to each group, and the average value of each group was randomly distributed so that the average value was evenly distributed. Test material (the embodiment compound and comparison compound) were used by dissolving them in 0.5% methylcellulose solution, and tegoprazan was used as a comparative compound. The prepared test material was orally administered to test animals at an amount of 3mg/5mL/kg, and 30 minutes later, inhalation anesthesia (3% isoflurane) was performed for 3 minutes using an inhalation anesthesia machine. After making a minimal incision in the upper abdomen about 1 cm below the left side of the sternum of an anesthetized test animal, the stomach was exposed, and the pyloric ring, which is the connection between the stomach and the duodenum, was ligated. The stomach and duodenum were restored to their original positions, and the laparotomy site was sutured well with surgical staplers. Histamine (7.5mg/2.5mL/head) was slowly administered subcutaneously to the back of the neck to promote gastric acid secretion. 3 hours after the surgery, the experimental animals were anesthetized with isoflurane and the stomach was removed through laparotomy. Gastric fluid obtained from the extracted stomach was transferred to a 15 mL conical tube, centrifuged at 5,000 rpm for 10 minutes, and only the supernatant was separated. 0.2 mL of the separated gastric fluid was diluted in 24.8 mL of distilled water to make a total dilution of 25 mL, and then 0.01N NaOH was added in small amounts to titrate until the pH reached 7. Efficacy was evaluated by calculating total acidity by combining the amount of gastric fluid and the amount of 0.01N NaOH required for titration.

$$Total\ Acid\ output\ (\mu Eq)$$
$$= \frac{mL\ of\ 0.01N\ NaOH\ x 0.01N\ x 1000\ x Total\ amount\ of\ the\ gastric\ juice}{Amount\ of\ the\ gastric\ juice\ taken\ for\ titration}$$

The measured result was shown in the following table 2.

[Table 2]

| Compound | gastric acid releasing suppression ability(% INH, Mean±SD) |
|---|---|
| Comparison compound | 100 ± 0.0 *** |
| Embodiment 1 | 99.7 ± 0.2 *** |
| Embodiment 2 | 26.8 ± 9.2 * |
| Embodiment 3 | 23.9 ± 3.3 * |
| Embodiment 4 | 100.0 ± 0.0 *** |
| Embodiment 5 | 12.7 ± 10.9 * |
| Embodiment 6 | 92.8 ± 4.8 *** |
| *, *** : p < 0.05, p < 0.001 (vs Excipient control) | |

As shown in the table 2, it was confirmed that the compounds of embodiments 1, 4, and 6 according to the present invention have the powerful gastric acid releasing suppression ability in the gastric acid secretion animal model.

**experimental example 3: gastric acid releasing suppression ability qualification test by the administration in the duodenum.**

A week before the test, test animals (rats) were purchased and acclimatized. After acclimatization, food was removed the day before the test and fasting was performed for 24 hours using a fasting plate. The weight of each individual was measured, weight ranking was sequentially assigned to each group, and the average value of each group was randomly distributed so that the average value was evenly distributed. Test material (the embodiment compound and comparison compound) were used by dissolving them in 0.5% methylcellulose solution, and tegoprazan was used as a comparative compound. Experimental animals were subjected to inhalation anesthesia (3% isoflurane) for 3 minutes using an inhalation anesthesia machine. After making a minimal incision in the upper abdomen about 1 cm below the left side of the sternum of an anesthetized test animal, the stomach was exposed, and the pyloric ring, which is the connection

between the stomach and the duodenum, was ligated. The prepared drug was administered into the duodenum in an amount of 1mg/5mL/kg, the stomach and duodenum were restored to their original positions, and the laparotomy site was sutured well with a surgical stapler. Histamine (7.5mg/2.5mL/head) was slowly administered subcutaneously to the back of the neck to promote gastric acid secretion. 3 hours after the surgery, the experimental animals were anesthetized with isoflurane and the stomach was removed through laparotomy. Gastric fluid obtained from the extracted stomach was transferred to a 15 mL conical tube, centrifuged at 5,000 rpm for 10 minutes, and only the supernatant was separated. 0.2 mL of the separated gastric fluid was diluted in 24.8 mL of distilled water to make a total dilution of 25 mL, and then 0.01N NaOH was added in small amounts to titrate until the pH reached 7. Efficacy was evaluated by calculating total acidity by combining the amount of gastric fluid and the amount of 0.01N NaOH required for titration.

$$Total\ Acid\ output\ (\mu Eq) = \frac{mL\ of\ 0.01N\ NaOH\ x 0.01N\ x 1000\ x Total\ amount\ of\ the\ gastric\ juice}{Amount\ of\ the\ gastric\ juice\ taken\ for\ titration}$$

The measured result was shown in the following table 3.

[Table 3]

| Compound | gastric acid releasing suppression ability(% INH, Mean±SD) |
|---|---|
| Comparison compound | 29.8 ± 9.1 |
| Embodiment 6 | 43.7 ± 8.7 * |
| * : p < 0.05 (vs Excipient control) | |

As shown in the table 3, the compound of embodiment 6 according to the present invention showed an improved ability to inhibit gastric acid secretion compared to the comparative compound when the drug was administered to the duodenum with a neutral pH. Therefore, the compound of embodiment 6 according to the present invention is expected to exert the same effect regardless of pH even after a meal or in various biological environments in which the pH in the stomach cavity is neutral.

## Claims

1. A benzimidazole derivative compound represented by Formula 1 or Formula 2, or a pharmaceutically acceptable salt thereof or a stereoisomer thereof:

[Formula 1]

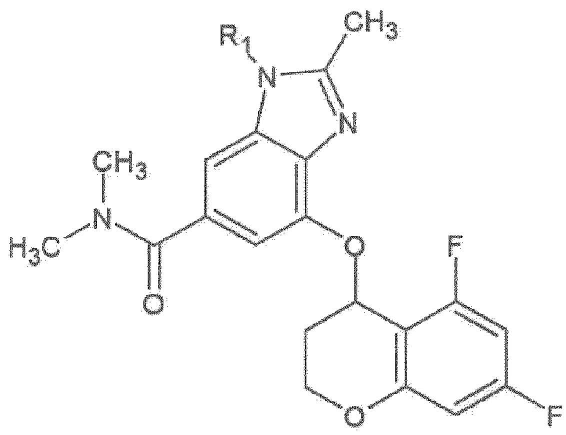

[Formula 2]

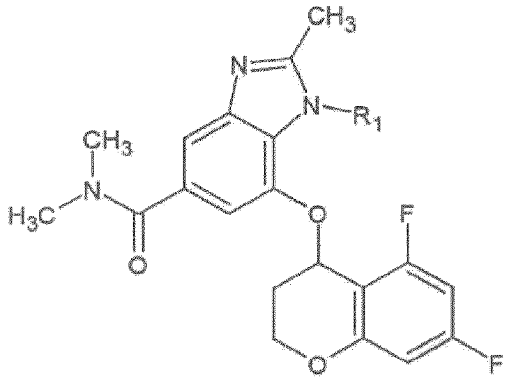

in the Formula above,

wherein $R_1$ is

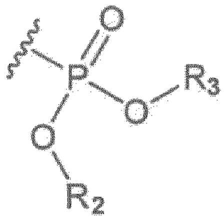 , 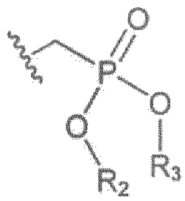 or 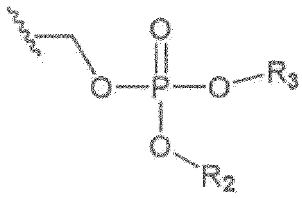 ,

wherein $R_2$ and $R_3$ are each independently hydrogen, alkyl or cycloalkyl.

**2.** The benzimidazole derivative compound, or a pharmaceutically acceptable salt thereof or a stereoisomer thereof according to claim 1, wherein
$R_2$ and $R_3$ are each independently hydrogen, C1-C7 alkyl or $C_3$-$C_7$ cycloalkyl.

**3.** The benzimidazole derivative compound, or a pharmaceutically acceptable salt thereof or a stereoisomer thereof according to claim 2, wherein
$R_2$ and $R_3$ are each independently hydrogen, or C1-C5 alkyl.

**4.** The benzimidazole derivative compound, or a pharmaceutically acceptable salt thereof or a stereoisomer thereof according to claim 1, wherein
the pharmaceutically acceptable salt is alkali metal or alkaline-earth metal salt selected from the group consisting of sodium, lithium, potassium, calcium and magnesium.

**5.** The benzimidazole derivative compound, or a pharmaceutically acceptable salt thereof or a stereoisomer thereof according to claim 4, wherein
the pharmaceutically acceptable salt is sodium salt.

**6.** The benzimidazole derivative compound, or a pharmaceutically acceptable salt thereof or a stereoisomer thereof according to claim 1, wherein
the benzimidazole derivative compound represented by Formula 1 or Formula 2 is selected from the following group:

diisopropyl(S)-(4-((5,7-difluorochroman-4-yl)oxy)-6-(dimethylcarbamoyl)-2-methyl-1H-benzo[d]imidazol-1-yl) phosphonate;
(S)-di-tert-butyl((4-((5,7-difluorochroman-4-yl)oxy)-6-(dimethylcarbamoyl)-2-methyl-1H-benzo[d]imidazol-1-yl) methyl) phosphate;
tert-butyl((4-(((S)-5,7-difluorochroman-4-yl)oxy)-6-(dimethylcarbamoyl)-2-methyl-1H-benzo[d]imidazol-1-yl) methyl)hydrogen phosphate;
(S)-(4-((5,7-difluorochroman-4-yl)oxy)-6-(dimethylcarbamoyl)-2-methyl-1H-benzo[d]imidazol-1-yl)methyl dihydrogen phosphate;
sodium(S)-tert-butyl ((4-((5,7-difluorochroman-4-yl)oxy)-6-(dimethylcarbamoyl)-2-methyl-1H-benzo[d]imidazol-1-yl)methyl) phosphate;
sodium(S)-(4-((5,7-difluorochroman-4-yl)oxy)-6-(dimethylcarbamoyl)-2-methyl-1H-benzo[d]imidazol-1-yl) methyl phosphate.

**7.** A pharmaceutical composition for the inhibition of gastric acid secretion comprising the benzimidazole derivative compound, or a pharmaceutically acceptable salt thereof or a stereoisomer thereof according to claim 1 to claim 6 as an active ingredient, with a pharmaceutically acceptable carrier.

**8.** A pharmaceutical composition for the preventing or treating diseases caused by disorders of gastric acid secretion comprising the benzimidazole derivative compound, or a pharmaceutically acceptable salt thereof or a stereoisomer thereof according to claim 1 to claim 6 as an active ingredient, with a pharmaceutically acceptable carrier.

**9.** The pharmaceutical composition of claim 8, wherein
the disease caused by disorders of gastric acid secretion is selected from the group consisting of gastrointestinal disease, gastroesophageal disease, gastroesophageal reflux disease (GERD), peptic ulcer, gastric ulcer, duodenal ulcer, NSAID-induced ulcer, gastritis, Helicobacter pylori infection, dyspepsia, functional dyspepsia, Zollinger-Ellison syndrome, non-erosive reflux disease(NERD), referred visceral pain, heartburn, nausea, esophagitis, dysphagia, drooling, airway disorders and asthma.

## Patentansprüche

**1.** Benzimidazolderivat-Verbindung, die mit Formel 1 oder Formel 2 wiedergegeben ist, oder ein pharmazeutisch akzeptables Salz davon oder ein Stereoisomer davon:

[Formel 1]

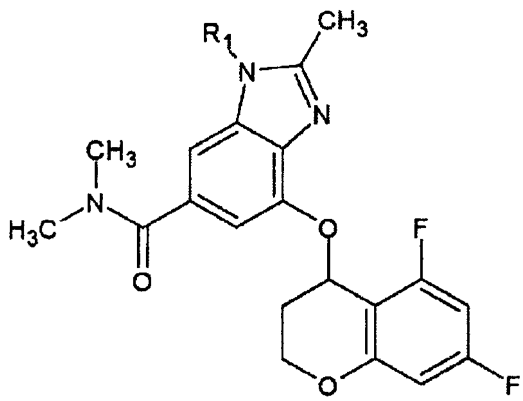

[Formel 2]

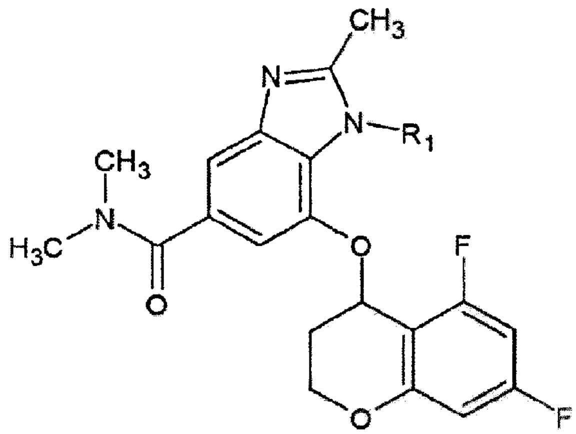

in der vorangehenden Formel,

in der $R_1$

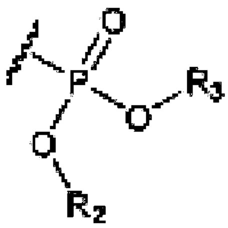,
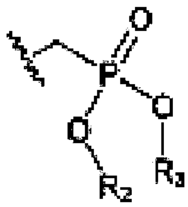
oder
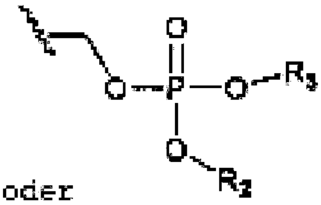,

ist,
wobei $R_2$ und $R_3$ jeweils unabhängig Wasserstoff, Alkyl oder Cycloalkyl sind.

**2.** Benzimidazolderivat-Verbindung oder ein pharmazeutisch akzeptables Salz davon oder ein Stereoisomer davon nach Anspruch 1, wobei
$R_2$ und $R_3$ jeweils unabhängig Wasserstoff, $C_1$-$C_7$-Alkyl oder $C_3$-$C_7$-Cycloalkyl sind.

**3.** Benzimidazolderivat-Verbindung oder ein pharmazeutisch akzeptables Salz davon oder ein Stereoisomer davon nach Anspruch 2, wobei
$R_2$ und $R_3$ jeweils unabhängig Wasserstoff oder $C_1$-$C_5$-Alkyl sind.

**4.** Benzimidazolderivat-Verbindung oder ein pharmazeutisch akzeptables Salz davon oder ein Stereoisomer davon nach Anspruch 1, wobei
das pharmazeutisch akzeptable Salz ein Alkalimetall- oder Erdalkalimetallsalz ist, ausgewählt aus der Gruppe bestehend aus Natrium, Lithium, Kalium, Calcium und Magnesium.

**5.** Benzimidazolderivat-Verbindung oder ein pharmazeutisch akzeptables Salz davon oder ein Stereoisomer davon nach Anspruch 4, wobei
das pharmazeutisch akzeptable Salz Natriumsalz ist.

**6.** Benzimidazolderivat-Verbindung oder ein pharmazeutisch akzeptables Salz davon oder ein Stereoisomer davon nach Anspruch 1, wobei
die Benzimidazolderivat-Verbindung, die mit Formel 1 oder Formel 2 wiedergegeben ist, aus der folgenden Gruppe ausgewählt ist:

Diisopropyl(S)-(4-((5,7-difluorchroman-4-yl) oxy)-6-(dimethylcarbamoyl)-2-methyl-1H-benzo[d]imidazol-1-yl) phosphonat;
(S)-Di-tert-butyl((4-((5,7-difluorchroman-4-yl)oxy)-6-(dimethylcarbamoyl)-2-methyl-1H-benzo[d]imidazol-1-yl)methyl)phosphat;
tert-Butyl((4-(((S)-5,7-difluorchroman-4-yl)oxy)-6-(dimethylcarbamoyl)-2-methyl-1H-benzo[d]imidazol-1-yl) methyl)wasserstoffphosphat;
(S)-(4-((5,7-Difluorchroman-4-yl)oxy)-6-(dimethylcarbamoyl)-2-methyl-1H-benzo[d]imidazol-1-yl)methyldiwasserstoffphosphat;
Natrium(S)-tert-butyl((4-((5,7-difluorchroman-4-yl)oxy)-6-(dimethylcarbamoyl)-2-methyl-1H-benzo[d]imidazol-1-yl)methyl)phosphat;
Natrium(S)-(4-((5,7-difluorchroman-4-yl)oxy)-6-(dimethylcarbamoyl)-2-methyl-1H-benzo[d]imidazol-1-yl)methylphosphat.

7. Pharmazeutische Zusammensetzung zum Hemmen der Sekretion von Magensäure, umfassend die Benzimidazolderivat-Verbindung oder ein pharmazeutisch akzeptables Salz davon oder ein Stereoisomer davon nach Anspruch 1 bis Anspruch 6 als Wirkstoff, mit einem pharmazeutisch akzeptablen Träger.

8. Pharmazeutische Zusammensetzung zum Verhindern oder Behandeln von Krankheiten, die durch Störungen bei der Sekretion von Magensäure verursacht werden, umfassend die Benzimidazolderivat-Verbindung oder ein pharmazeutisch akzeptables Salz davon oder ein Stereoisomer davon nach Anspruch 1 bis Anspruch 6 als Wirkstoff, mit einem pharmazeutisch akzeptablen Träger.

9. Pharmazeutische Zusammensetzung nach Anspruch 8, wobei
die Krankheit, die durch Störungen bei der Sekretion von Magensäure verursacht wird, ausgewählt ist aus der Gruppe, bestehend aus Magen-Darm-Erkrankung, gastroösophagealer Erkrankung, gastroösophagealer Refluxkrankheit (GERD), peptischem Geschwür des Magens, Magengeschwür, Zwölffingerdarmgeschwür, NSAID-induziertem Ulkus, Gastritis, Helicobacter-pylori-Infektion, Dyspepsie, funktioneller Dyspepsie, Zollinger-Ellison-Syndrom, nicht-erosiver Refluxkrankheit (NERD), ausstrahlenden viszeralen Schmerzen, Sodbrennen, Übelkeit, Ösophagitis, Dysphagie, Speichelfluss, Atemwegserkrankungen und Asthma.

## Revendications

1. Composé dérivé du benzimidazole représenté par la formule 1 ou la formule 2 ou un de ses sels pharmaceutiquement acceptables ou un de ses stéréosimères :

[Formule 1]

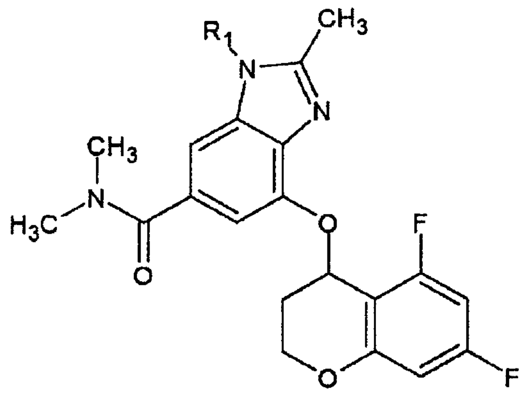

[Formule 2]

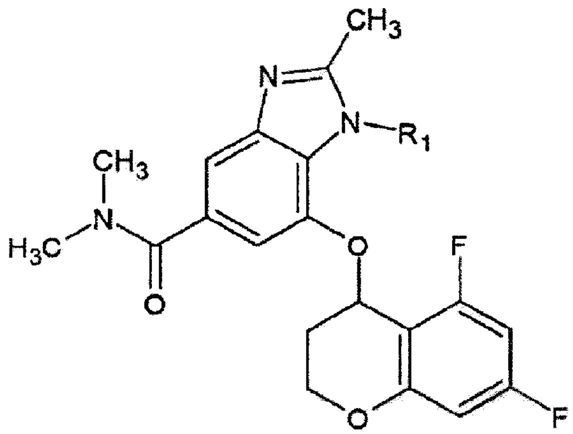

dans la formule ci-dessus,

où $R_1$ est

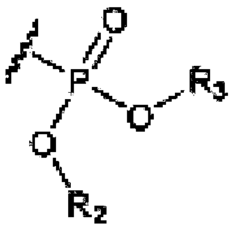 , 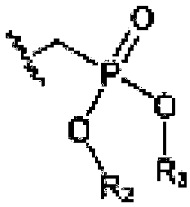 ou 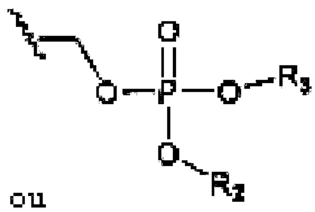 ,

où $R_2$ et $R_3$ sont chacun indépendamment un hydrogène, un alkyle ou un cycloalkyle,

**2.** Composé dérivé du benzimidazole ou un de ses sels pharmaceutiquement acceptables ou un de ses stéréoisomères selon la revendication 1, dans lequel
$R_2$ et $R_3$ sont chacun indépendamment un hydrogène, un alkyle en $C_1$ à $C_7$ ou un cycloalkyle en $C_3$ à $C_7$.

**3.** Composé dérivé du benzimidazole ou un de ses sels pharmaceutiquement acceptables ou un de ses stéréoisomères selon la revendication 2, dans lequel
$R_2$ et $R_3$ sont chacun indépendamment un hydrogène ou un alkyle en $C_1$ à $C_5$.

**4.** Composé dérivé du benzimidazole ou un de ses sels pharmaceutiquement acceptables ou un de ses stéréoisomères selon la revendication 1, dans lequel
le sel pharmaceutiquement acceptable est un sel de métal alcalin ou alcalino-terreux choisi dans le groupe constitué par le sodium, le lithium, le potassium, le calcium, et le magnésium.

**5.** Composé dérivé du benzimidazole ou un de ses sels pharmaceutiquement acceptables ou un de ses stéréoisomères selon la revendication 4, dans lequel
le sel pharmaceutiquement acceptable est un sel de sodium.

**6.** Composé dérivé du benzimidazole ou un de ses sels pharmaceutiquement acceptables ou un de ses stéréoisomères selon la revendication 1, dans lequel
le composé dérivé du benzimidazole représenté par la formule 1 ou la formule 2 est choisi dans le groupe suivant :

le (S)-(4-((5,7-difluorochroman-4-yl)oxy)-6-(diméthylcarbamoyl)-2-méthyl-1H-benzo[d]imidazole-1-yl)phosphonate de diisopropyle ;
le (S)-di-tert-butyl((4-((5,7-difluorochroman-4-yl)oxy)-6-(diméthylcarbamoyl)-2-méthyl-1H-benzo[d]imidazole-1-yl)méthyl)phosphate ;
le tert-butyl((4-(((S)-5,7-difluorochroman-4-yl)oxy)-6-(diméthylcarbamoyl)-2-méthyl-1H-benzo[d]imidazole-1-yl)méthyl)hydrogène phosphate ;
le (S)-(4-(5,7-difluorochroman-4-yl)oxy)-6-(diméthylcarbamoyl)-2-méthyl-1H-benzo[d]imidazole-1-yl)méthyl dihydrogène phosphate ;
le (S)-tert-butyl((4-((5,7-difluorochroman-4-yl)oxy-6-(diméthylcarbamoyl)-2-méthyl-1H-benzo[d]imidazole-1-yl) méthyl) phosphate de sodium ;
le (S)-(4-((5,7-difluorochroman-4-yl)oxy)-6-(diméthylcarbamoyl)-2-méthyl-1H-benzo [d]imidazole-1-yl)méthyle phosphate de sodium.

**7.** Composition pharmaceutique pour l'inhibition de la sécrétion acide gastrique comprenant le composé dérivé du benzimidazole ou un de ses sels pharmaceutiquement acceptables ou un de ses stéréoisomères selon la revendication 1 à la revendication 6 comme ingrédient actif, avec un véhicule pharmaceutiquement acceptable.

**8.** Composition pharmaceutique pour la prévention ou le traitement de maladies provoquées par des troubles de la sécrétion acide gastrique comprenant le composé dérivé du benzimidzole ou un de ses sels pharmaceutiquement acceptables ou un de ses stéréoisomères selon la revendication 1 à la revendication 6 comme ingrédient actif, avec un véhicule pharmaceutiquement acceptable.

**9.** Composition pharmaceutique selon la revendication 8, dans laquelle
la maladie provoquée par les troubles de sécrétion d'acide gastrique est choisie dans le groupe constitué par la maladie gastrointestinale, la maladie gastroœsophagienne, la maladie du reflux gastroœsophagien (GERD), l'ulcère peptique, l'ulcère gastrique, l'ulcère duodénal, l'ulcère induit par NSAID, la gastrite, l'infection par Helicobacter pylori, la dyspepsie, la dyspepsie fonctionnelle, le syndrome de Zollinger-Ellison, la maladie du reflux non érosif (NERD), la douleur viscérale rapportée, la brûlure du cœur, la nausée, l'œsophagite, la dysphagie, la salivation, les troubles des voies aériennes, et l'asthme.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2006134460 A1
- KR 20120052269 A
- KR 101088247 B1
- KR 101829706 B1
- WO 2020210878 A1


### Non-patent literature cited in the description


- **ABET VALENTINA et al.** Prodrug approach: An overview of recent cases. *European Journal of Medicinal Chemistry*, 2016, vol. 127, 810-827